# EUROPEAN PATENT APPLICATION

(11) **EP 4 495 230 A1**
(43) Date of publication of application: **22.01.2025**
(21) Application number: 23769949.1
(22) Date of filing: 15.05.2023
(51) Int. Cl.: C12N 5/0775, A61P 17/02, A61K 35/28, A61K 45/06, G01N 33/68, C12Q 1/6888, G16B 45/00

(54) **MESENCHYMAL STEM CELL WOUND REPAIR DOMINANT FUNCTIONAL CLUSTER, IDENTIFICATION THEREOF, AND APPLICATION**

(30) Priority: 14.03.2022 CN 202210249611
(71) Applicant: FUJIAN MEDICAL UNIVERSITY UNION HOSPITAL, Fuzhou, Fujian 350001 (CN)
(72) Inventor: CHEN, Xiaosong, Fuzhou, Fujian 350001 (CN)
(74) Representative: Michalski Hüttermann & Partner Patentanwälte mbB
(86) International application number: PCT/CN2023/094202
(87) International publication number: WO 2023/174447

(57) **Abstract**

Disclosed are a mesenchymal stem cell wound repair dominant functional cluster (Hr-MSC), identification thereof, and an application. The mesenchymal stem cell wound repair dominant functional cluster has a biomarker combination. The wound repair dominant functional cluster can efficiently promote the healing of wounds, can be used for accelerating clinical healing of various acute and chronic wounds, and can be used as intervention in various stages of wound healing, so as to achieve the objective of accelerating scar-free healing of a wound; also, ample data support and technical support are provided for research and development of stem cell innovative drugs, and clinical application of stem cell innovative drugs is accelerated, customized development of illness-shortening "stem cell innovative drugs" that improve the quality of healing of a wound are facilitated, and finally a scientific and sensible stem cell application standardized system is established.

## Description

### CROSS-REFERENCE TO RELATED APPLICATIONS

This application is a continuation application of PCT/CN2023/094202, filed on May 15, 2023, which claims priority from Chinese patent application CN202210249611.4, filed on March 14, 2022, the entire contents of which are hereby incorporated by reference.

### TECHNICAL FIELD

The present disclosure belongs to the field of biology, and particularly relates to a wound repair-dominant functional cluster of mesenchymal stem cells, identification thereof, and use thereof.

### BACKGROUND

Human umbilical cord mesenchymal stem cells (HUCMSCs) are one of the ideal seed cells and have shown good application prospects in the field of tissue damage repair. However, in the review of many studies, it has been found that the therapeutic effect of stem cells varies to different degrees, and the implantation efficiency and integration rate are relatively low, which is primarily due to the functional variation and cell heterogeneity of mesenchymal stem cells (MSCs).

Single-cell transcriptome sequencing technology can realize gene reproduction in a single cell, which is of great significance for discovering cell heterogeneity, revealing cell functions, describing relationships across omes, establishing a complete map, and the like.

Single-cell multi-omics ATAC + GEX (assay for transposase-accessible chromatin and gene expression) technology can simultaneously detect RNA and chromatin accessibility in a single cell, more deeply characterize cell types of different lineages, capture cell heterogeneity, deduce the full picture of a molecular regulation network combined with an epigenetic expression profile and a gene expression profile, obtain more real and systematic molecular change information, and is a brand new direction for a panoramic analysis of the spatiotemporal heterogeneity of umbilical cord mesenchymal stem cells.

Flow cytometry (FCM) is a rapidly developing biomedical analysis technology, and is a novel high-tech cell analysis technology that integrates laser technology, photoelectric measurement technology, computer technology, hydrodynamics, cellular immunofluorescence chemistry technology, and monoclonal antibody technology. A flow cytometer is an instrument established using flow cytometry. It allows cells or particles to flow in a fluid stream, passing through an incident beam one by one, and records scattered light and various fluorescent signals with a highly sensitive detector, thereby allowing multi-parameter analysis of the particles, including parameters such as particle shape and size, cell cycle, intracellular cytokines, bacterial surface antigens, cellular DNA content, and the like. The particles detected by FCM are generally living cells, and various biological information inside the cells can be obtained by exciting the intensity and color of the fluorescent substances labeled on the cells and the intensity of scattered light by a laser light source. Additionally, FCM can also be used for cell sorting, which can be done according to certain properties of the detected cells, and the cells can be used for culture if the sorting is carried out under sterile conditions.

### SUMMARY

The technical problem to be solved in the present disclosure is for overcoming the defects of inconsistent repairing efficacy, poor repeatability, and low implantation rate of mesenchymal stem cells for wound repair in the prior art, and provided are a wound repair-dominant functional cell cluster of mesenchymal stem cells, identification thereof, and use thereof. The wound repair-dominant functional cell clusters of the present disclosure have the same functional characteristics and good repeatability, and can be used for accelerating wound healing.

The inventor discussed and demonstrated the heterogeneity of mesenchymal stem cells such as umbilical cord mesenchymal stem cells through experiments. Utilizing single-cell multi-omics ATAC + GEX and spatial transcriptome sequencing, the inventor applied single-cell multimodal omics to comprehensively map the heterogeneity of umbilical cord mesenchymal stem cells in multiple dimensions. Combining the inventor's research findings in clinical practice and wound repair, a dominant functional cluster related to wound healing was ultimately selected using flow cytometry sorting technology, and the cluster was amplified, identified, and preliminarily validated in animal models.

The present disclosure solves the above-mentioned technical problem by the following technical solutions.

A first aspect of the present disclosure provides a biomarker combination for a wound repair-dominant functional cell, comprising CD29, CD142, and CYR61.

CD29 is also called ITGB1, and CD142 is also called F3.

A second aspect of the present disclosure provides a biomarker combination for a wound repair-dominant functional cell, comprising CD29, CD142, and S100A9.

In the present disclosure, the wound refers to the damage caused by an external injury factor on the normal skin, so that the skin function is impaired. The wound includes partial loss of normal tissues, partial necrosis of tissue cells, and stress reactions in some tissues.

In the present disclosure, the biomarker combination may further comprise one or more biomarkers selected from: DCN, TGFB1, COL3A1, COLIA2, and COLIA1.

In the present disclosure, the biomarker combination may further comprise the following biomarkers: DCN, TGFB1, COL3A1, COLIA2, and COLIA1.

In the present disclosure, the biomarker combination may further comprise one or more biomarkers selected from: DCN, TGFB1, COL3A1, COLIA2, COLIA1, NEAT1, TMP1, GFBP4, SAT1, TGM2, LUM, SERPNF1, MXD4, FOS, ACTA2, HTRA3, HTRA1, PNRC1, APOE, and CDKN1 C.

In the present disclosure, the biomarker combination may further comprise the following biomarkers: DCN, TGFB1, COL3A1, COLIA2, COLIA1, NEAT1, TMP1, GFBP4, SAT1, TGM2, LUM, SERPNF1, MXD4, FOS, ACTA2, HTRA3, HTRA1, PNRC1, APOE, and CDKN1C.

A third aspect of the present disclosure provides a wound repair-dominant functional cell formulation, comprising a stem cell positive for CD29, CD142, and CYR61.

A fourth aspect of the present disclosure provides a wound repair-dominant functional cell formulation, comprising a stem cell positive for CD29, CD142, and S100A9.

In the present disclosure, preferably, the stem cell is a mesenchymal stem cell.

In the present disclosure, preferably, the stem cell expresses one or more biomarkers selected from DCN, TGFB1, COL3A1, COLIA2, and COLIA1.

In the present disclosure, more preferably, the stem cell expresses one or more biomarkers selected from NEAT1, TMP1, GFBP4, SAT1, TGM2, LUM, SERPNF1, MXD4, FOS, ACTA2, HTRA3, HTRA1, PNRC1, APOE, and CDKN1C.

A fifth aspect of the present disclosure provides a cell cluster, comprising a stem cell positive for CD29, CD142, and CYR61; the proportion of the stem cell in the cell cluster is at least 90%.

A sixth aspect of the present disclosure provides a cell cluster, comprising a stem cell positive for CD29, CD142, and S100A9; the proportion of the stem cell in the cell cluster is at least 90%.

In the present disclosure, the positive expression ratio of the biomarkers in the cell cluster is preferably at least 92%, at least 95%, at least 97%, at least 98%, at least 99%, or at least 100%.

In the present disclosure, the stem cell is preferably as described in the third or fourth aspect.

A seventh aspect of the present disclosure provides a pharmaceutical composition, comprising a stem cell and a pharmaceutically acceptable carrier and/or excipient; the stem cell is as described in the third or fourth aspect.

An eighth aspect of the present disclosure provides a pharmaceutical combination, comprising a first therapeutic agent and a second therapeutic agent; the first therapeutic agent comprises the pharmaceutical composition according to the seventh aspect.

In some embodiments of the present disclosure, the second therapeutic agent is selected from one or more of an immunosuppressant, an analgesic, and an anti-infective agent.

A ninth aspect of the present disclosure provides use of the biomarker combination according to the first or second aspect, the wound repair-dominant functional cell formulation according to the third or fourth aspect, the cell cluster according to the fifth or sixth aspect, the pharmaceutical composition according to the seventh aspect, or the pharmaceutical combination according to the eighth aspect in the manufacture of a formulation for promoting wound repair.

A tenth aspect of the present disclosure provides a method for promoting wound repair, the method comprising administering to a subject in need the wound repair-dominant functional cell formulation according to the third or fourth aspect, the cell cluster according to the fifth or sixth aspect, the pharmaceutical composition according to the seventh aspect, or the pharmaceutical combination according to the eighth aspect.

An eleventh aspect of the present disclosure provides a method for identifying a wound repair-dominant functional cell cluster for a non-diagnostic purpose, the method comprising:
(1) performing molecular typing on test cells based on the single-cell multimodal omics technology to obtain a target candidate cell cluster; and
(2) performing characteristic gene display and sorting on the target candidate cell cluster obtained in (1) to obtain a cell cluster comprising characteristic genes, the cell cluster being a wound repair-dominant functional cell cluster;
the characteristic genes expressing the biomarker combination according to the first or second aspect.

In the present disclosure, the non-diagnostic purpose means that the wound repair-dominant functional cell cluster obtained according to the identification method cannot be used as a single clinical index of wound repair; the wound repair still needs the experience and professional analysis and judgment of clinical medical personnel, and the wound repair-dominant functional cell cluster in the present disclosure is an important reference index.

In some embodiments of the present disclosure, the positive expression ratio of the biomarker combination according to the first or second aspect in the cell cluster is at least 90%, e.g., at least 92%, at least 95%, at least 97%, at least 98%, at least 99%, or at least 100%.

In some embodiments of the present disclosure, the single-cell multimodal omics technology may be conventional in the art, e.g., including the single-cell multi-omics technology such as the ATAC + GEX technology.

In some embodiments of the present disclosure, the cell is a mesenchymal stem cell, such as an umbilical cord mesenchymal stem cell.

In the present disclosure, the mesenchymal stem cell is a wound repair-dominant functional cluster (Hr-MSC) of human umbilical cord mesenchymal stem cells, and this concept was first proposed by the inventor.

In some embodiments of the present disclosure, (1) is followed by (1-1): obtaining positioning information of the target candidate cell cluster through spatial transcriptomics, the positioning information comprising an enrichment condition and a positioning condition.

A twelfth aspect of the present disclosure provides a system for identifying a wound repair-dominant functional cluster cell, the system comprising a typing module and a gene display module;

where the typing module performs molecular typing on test cells by the single-cell multimodal omics technology to obtain a target candidate cell cluster; the gene display module performs characteristic gene display and sorting on the target candidate cell cluster obtained by the typing module to obtain a functional cell expressing characteristic genes, and the functional cell is the wound repair-dominant functional cluster cell;

the characteristic genes comprise/express the biomarker combination according to the first or second aspect.

In some embodiments of the present disclosure, the positive expression ratio of the marker combination according to the first or second aspect in the functional cell cluster is at least 90%, e.g., at least 92%, at least 95%, at least 97%, at least 98%, at least 99%, or at least 100%.

A thirteenth aspect of the present disclosure provides a kit for identifying the biomarker combination according to the first or second aspect, or the cell cluster according to the fifth or sixth aspect.

In some embodiments of the present disclosure, the kit comprises a reagent for detecting the expression level of the biomarker combination according to the first or second aspect. The reagent is, for example, a reagent for detecting the protein expression level of markers, or a reagent for detecting the gene or transcription level of markers.

In some embodiments of the present disclosure, the kit comprises the marker combination according to the first or second aspect.

The cell formulation of the present disclosure can be used for preparing a material for regenerative medicine.

In the present disclosure, the stem cell is a cell that has the ability to differentiate into various cells and the ability to self-renew, including an embryonic stem cell, a mesenchymal stem cell, an umbilical cord stem cell, an umbilical cord blood stem cell, an adipose-derived stem cell, a pluripotent stem cell, an induced pluripotent stem cell, and the like.

In the present disclosure, the positive means that cells can be sorted into positive cells by FACS, or the expression of corresponding markers can be detected by RT-PCR.

Based on the clustering and characteristic gene display results of the single-cell multimodal omics, the dominant functional cluster Hr-MSC with the function of promoting wound healing is screened in the present disclosure. Comprehensive preliminary screening is performed according to the expression of marker genes in each cluster, the functional enrichment of each cluster, and the expression of transcription factors in each cluster, and combined with databases such as Cellmarker, PanglaoDB, EBI, and the like. Where the present disclosure is the first to apply the single-cell multi-omics ATAC + GEX technology and the spatial transcriptomics technology to the research on the spatial heterogeneity of umbilical cord Wharton's jelly mesenchymal stem cells. The preliminary screening comprises: positioning: subcellular positioning of cluster marker genes and tissue positioning of immunofluorescence; and quantification: horizontal comparison of the proportions of clusters in different generations, different individuals, and different genders.

On the basis of the general knowledge in the art, the above preferred conditions can be combined arbitrarily to obtain preferred embodiments of the present disclosure.

The reagents and starting materials used in the present disclosure are commercially available.

The positive and progressive effects of the present disclosure are as follows:

The wound repair-dominant functional cluster (Hr-MSC) of the present disclosure can efficiently promote the healing of wounds (including various acute wounds, chronic refractory wounds, etc.), can be used for accelerating clinical healing of various acute and chronic wounds, and can be used as an intervention in various stages of wound healing, so as to achieve the objective of accelerating scar-free healing of wounds; moreover, sufficient data support and technical support are provided for the research and development of innovative stem cell drugs, and the clinical application of innovative stem cell drugs is accelerated, thereby facilitating the customized development of illness-shortening "innovative stem cell drugs" that improve the quality of wound healing, and finally establishing a scientific and reasonable stem cell application standardized system.

### BRIEF DESCRIPTION OF THE DRAWINGS

FIG. 1 is a schematic diagram showing the clustering results of umbilical cord mesenchymal stem cells.
FIGs. 2A-2D are schematic diagrams showing the heatmap results of marker genes of Hr-MSC.
FIGs. 3A and 3B are schematic diagrams showing the expression levels of marker genes of Hr-MSC.
FIG. 4 shows the functional enrichment analysis and the gene heatmap display for 13 clusters.
FIG. 5 is a UMAP clustering plot of four different WJ-MSC clusters.
FIG. 6 is a schematic diagram showing the clustering results of umbilical cord stem cells.
FIG. 7 is a schematic diagram showing the sorting results of CD29⁺/CD142⁺/S100A9⁺ Hr-MSC.
FIG. 8 is a schematic diagram showing the results of wound healing efficiency in Example 6.
FIG. 9 is a schematic diagram showing the migration capability of CD29⁺/CD142⁺/S100A9⁺ Hr-MSC on HDF and HUCV.
FIG. 10 is a schematic diagram showing the wound healing on the skin of zebrafish.
FIG. 11 shows a system 21 for identifying a wound repair-dominant functional cell cluster, which comprises a typing module 11 and a gene display module 21.

### DETAILED DESCRIPTION

The present disclosure is further illustrated by the following examples; however, these examples should not be construed as limiting the present disclosure. Experimental procedures without specified conditions in the following examples were conducted in accordance with conventional procedures and conditions, or in accordance with the manufacturer's manual.

### Example 1: Clustering of Umbilical Cord Stem Cells

In this example, the umbilical cord mesenchymal stem cells were subjected to sequencing analysis by using the single-cell transcriptomics to primarily divide the umbilical cord stem cells into different clusters at a single-cell level, and functional annotation was performed on each cluster by using bioinformatics analysis to determine a target candidate cluster.
1. Sample: human umbilical cord Wharton's jelly tissues;
   pregnant women who were at 37-40 weeks of gestation and delivered were enrolled, and the umbilical cords of newborns were selected for the experiment and served as the source of subsequent P0 and P3 generations of umbilical cord mesenchymal stem cell tissues, number: PUM-A1-000152;
   cells of 6 samples (huc_1P0, huc_1P3, huc_2P0, huc_2P3, huc_3P0, and huc_3P3) were selected for downstream analysis.
2. Instrument: 10× Chromium;
3. Parameters:
   (1) 8 channels were provided for each operation, with each channel capable of collecting 100-10000 cells;
   (2) the cell capture efficiency was about 65%;
   (3) the doublet ratio in the sequencing results was 0.9%/1000 cells; and
   (4) no restrictions were placed on cell size.
4. Sequencing and subsequent data analysis were performed using the Illumina NovaSeq sequencing platform: barcodes, UMIs, and RNA sequences were extracted from the original sequencing data, and a human reference genome was selected for alignment to obtain a feature-barcode matrix. The subsequent analysis was as follows:
   (1) The data were filtered for gene counts, UMI counts, and mitochondria, and a regularized negative binomial model for gene expression was constructed using sctransform for data normalization, followed by PCA dimensionality reduction, with the number of PCAs determined according to a scree plot;
   (2) Dimensionality reduction algorithms such as t-SNE and UMAP were used to transform the bioinformatics results obtained from the sequencing method into biological results, determining the heterogeneity of cell types in different individuals and regions, and visualization was performed to determine the heterogeneity of cell types in different individuals and obtain the typing results, thereby obtaining 13 clusters (Cluster 0, Cluster 1, Cluster 2, Cluster 3, Cluster 4, Cluster 5, Cluster 6, Cluster 7, Cluster 8, Cluster 9, Cluster 10, Cluster 11, and Cluster 12);
   (3) A FindClusters function was used to perform differential gene expression analysis on the clustering results, and the characteristic marker genes of the clusters were displayed in various forms, with Cluster 6 and Cluster 9 (i.e., Hr-MCS) being screened as potentially having similar functions according to the results;
   (4) Differentially expressed genes of each cluster in the Hr-MCS were subjected to analyses such as GO and KEGG enrichment analysis, protein interaction network analysis, and transcription factor annotation analysis to determine signaling pathways affected by the enrichment of upregulated genes in the Hr-MSC, speculating on the possible functions.

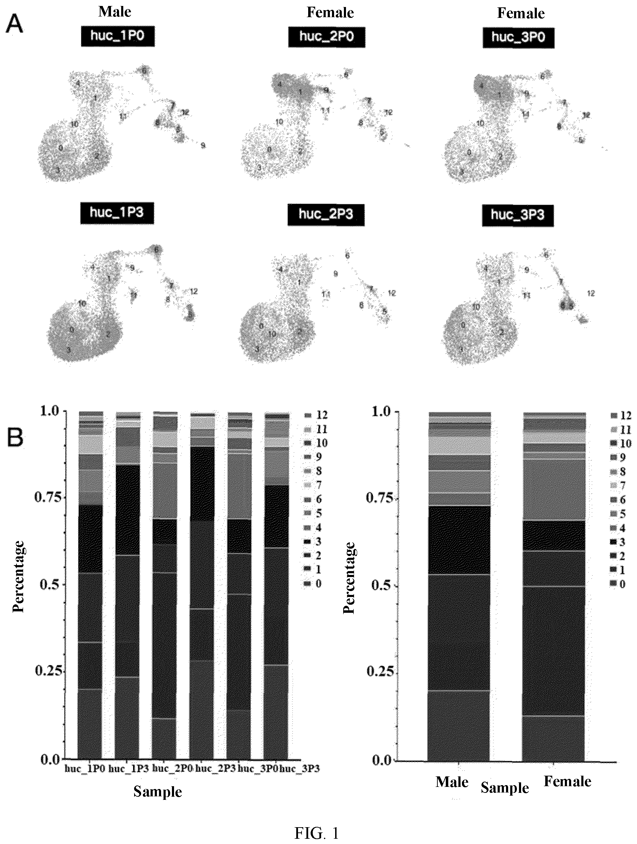

As shown in A and B of FIG. 1, the umbilical cord mesenchymal stem cells exhibited limited heterogeneity, and it was preliminarily determined that the composition of cells from different individuals, generations, and genders was consistent; however, the proportions of cells from different individuals, generations, and genders varied; and the genes expressed by the cells from different individuals, generations, and genders also varied.

### Example 2: Marker Gene Analysis of Hr-MSC

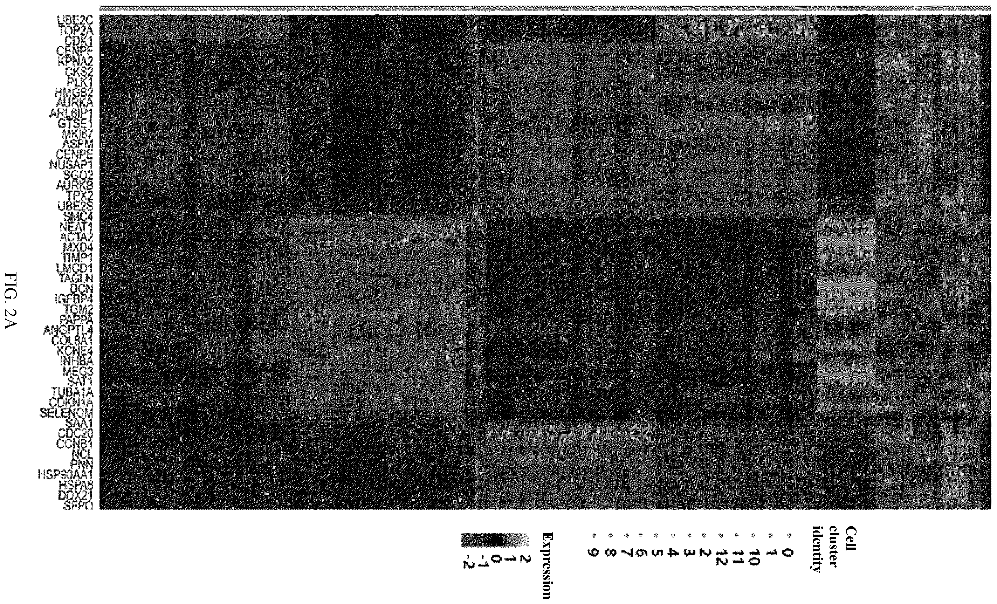

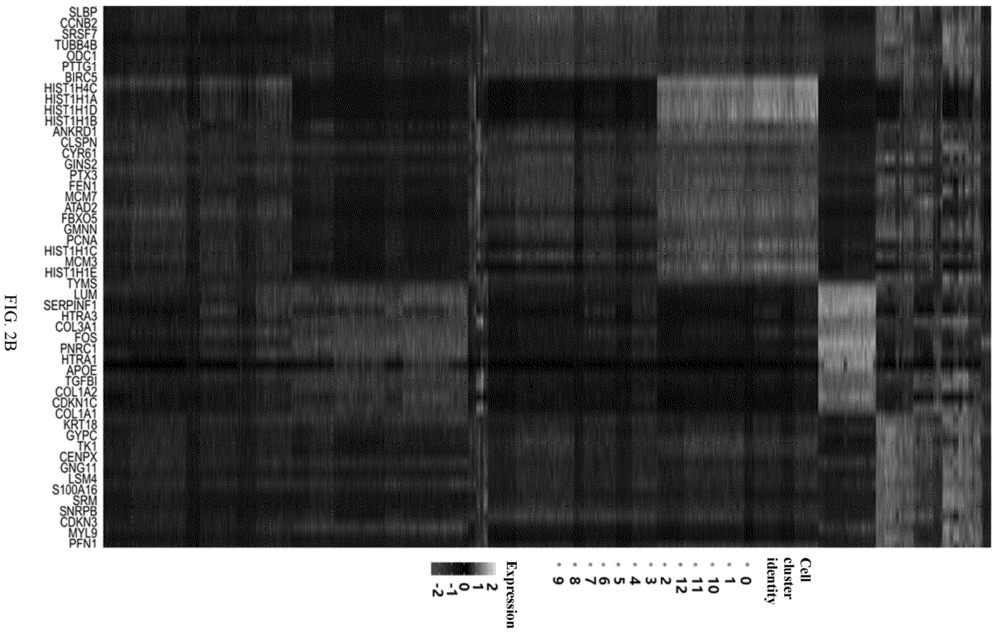

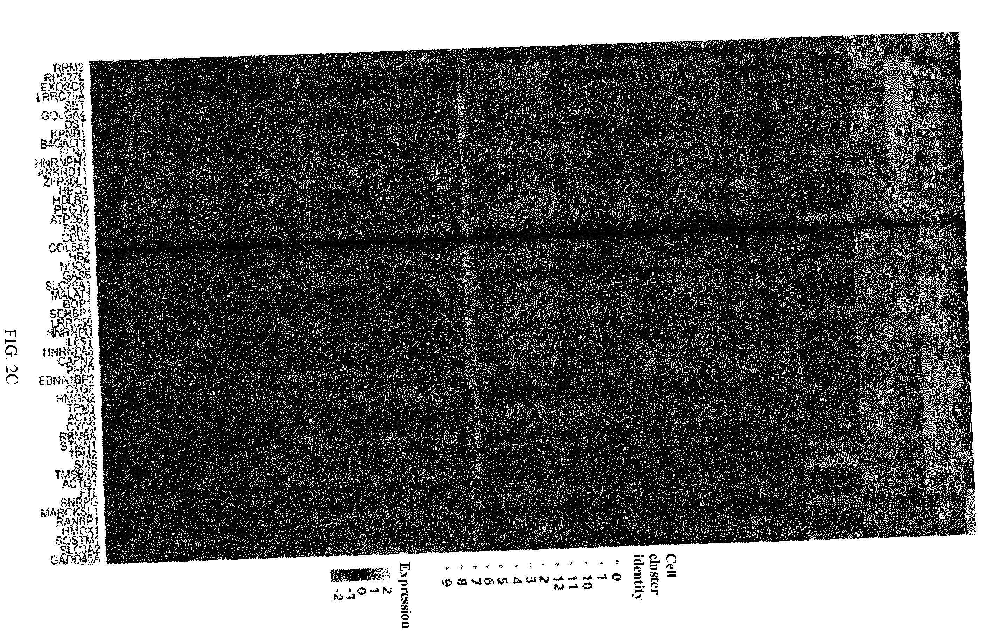

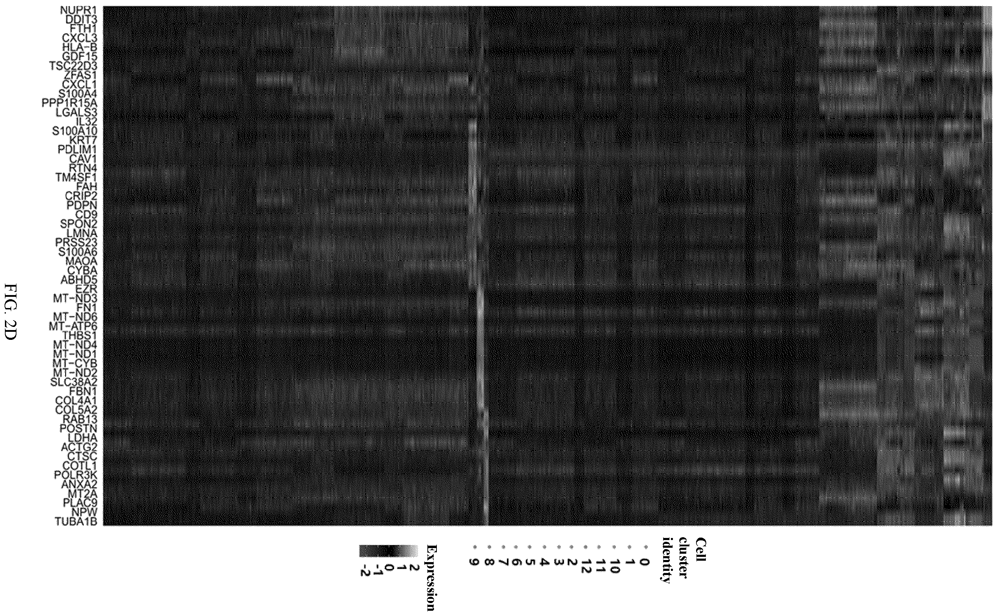

In this example, gene expression heatmaps were created for the marker genes of the various clusters of umbilical cord stem cells obtained in Example 1. The results are shown in FIGs. 2A-2D.

The marker genes in FIGs. 2A-2D include: UBE2C, TOP2A, CDK1, CENPF, KPNA2, CKS2, PLK1, HMGB2, AURKA, ARL6IP1, GTSE1, MKI67, ASPM, CENPE, NUSAP1, SGO2, AURKB, TPX2, UBE2S, SMC4, NEAT1, ACTA2, MXD4, TIMP1, LMCD1, TAGLN, DCN, IGFBP4, TGM2, PAPPA, ANGPTL4, COL8A1, KCNE4, INHBA, MEG3, SAT1, TUBA1A, CDKN1A, SELENOM, SAA1, CDC20, CCNB1, NCL, PNN, HSP90AA1, HSPA8, DDX21, SFPQ, SLBP, CCNB2, SRSF7, TUBB4B, ODC1, PTTG1, BIRC5, HIST1H4C, HIST1H1A, HIST1H1D, HIST1H1B, ANKRD1, CLSPN, CYR61, GINS2, PTX3, FEN1, MCM7, ATAD2, FBXOS, GMNN, PCNA, HIST1H1C, MCM3, HIST1H1E, TYMS, LUM, SERPINF1, HTRA3, COL3A1, FOS, PNRC1, HTRA1, APOE, TGFBI, COL1A2, CDKN1C, COL1A1, KRT18, GYPC, TK1, CENPX, GNG11, LSM4, S100A16, SRM, SNRPB, CDKN3, MYL9, PFN1, RRM2, RPS27L, EXOSC8, LRRC75A, SET, GOLGA4, DST, KPNB1, B4GALT1, FLNA, HNRNPH1, ANKRD11, ZFP36L1, HEG1, HDLBP, PEG10, ATP2B1, PAK2, CDV3, COL5A1, HBZ, NUDC, GAS6, SLC20A1, MALAT1, BOP1, SERBP1, LRRC59, HNRNPU, IL6ST, HNRNPA3, CAPN2, PFKP, EBNA1BP2, CTGF, HMGN2, TPM1, ACTB, CYCS, RBM8A, STMN1, TPM2, SMS, TMSB4X, ACTG1, FTL, SNRPG, MARCKSL1, RANBP1, HMOX1, SQSTM1, SLC3A2, GADD45A, NUPR1, DDIT3, FTH1, CXCL3, HLA-B, GDF15, TSC22D3, ZFAS1, CXCL1, S100A4, PPP1R15A, LGALS3, IL32, S100A10, KRT7, PDLIM1, CAV1, RTN4, TM4SF1, FAH, CRIP2, PDPN, CD9, SPON2, LMNA, PRSS23, S100A6, MAOA, CYBA, ABHD5, EZR, MT-ND3, FN1, MT-ND6, MT-ATP6, THBS1, MT-ND4, MT-ND1, MT-CYB, MT-ND2, SLC38A2, FBN1, COL4A1, COL5A2, RAB13, POSTN, LDHA, ACTG2, CTSC, COTL1, POLR3K, ANXA2, MT2A, PLAC9, NPW, and TUBA1B.

The cluster classification and the top 4 or top 5 marker genes are shown in Table 1.

**Table 1. Cluster classification and marker genes thereof**

| Cluster classification | Marker genes of each cluster (top 4 or top 5) |
|---|---|
| Cluster 0 | UBE2C; TOP2A; CDK1; CENPF; KPNA2 |
| Cluster 1 | NEAT1; ACTA2; MXD4; TIMP1; LMCD1 |
| Cluster 2 | CDC20; CCNB1; NCL; PNN; HSP90AA1 |
| Cluster 3 | HIST1H4C; HIST1H4A; HIST1H4D; HIST1H4B; ANKRD1 |
| Cluster 4 | DCN; SAT1; NEAT1; TGM2; TIMP1 |
| Cluster 5 | UBE2S; CKS2; TUBB4B; KRT18; GYPC |
| Cluster 6 | LRRC75A; SET; GOLGA4; MKI67; DST |
| Cluster 7 | HBZ; NUDC; ACTA2; GAS6; SLC20A1 |
| Cluster 8 | ACTA2; TAGLN; UBE2C; HMGN2 |
| Cluster 9 | HMOX1; SQSTM1; SLC3A2; GADD45A; NUPR1 |
| Cluster 10 | S100A10; KRT7; PDLIM1; CAV1; RTN4 |
| Cluster 11 | MT-ND3; FN1; MT-ND6; MALAT1; FLNA |
| Cluster 12 | RAB13; ACTA2; POSTN; LDHA; ACTG2 |

### Example 3: Expression Levels of Marker Genes of Hr-MSC

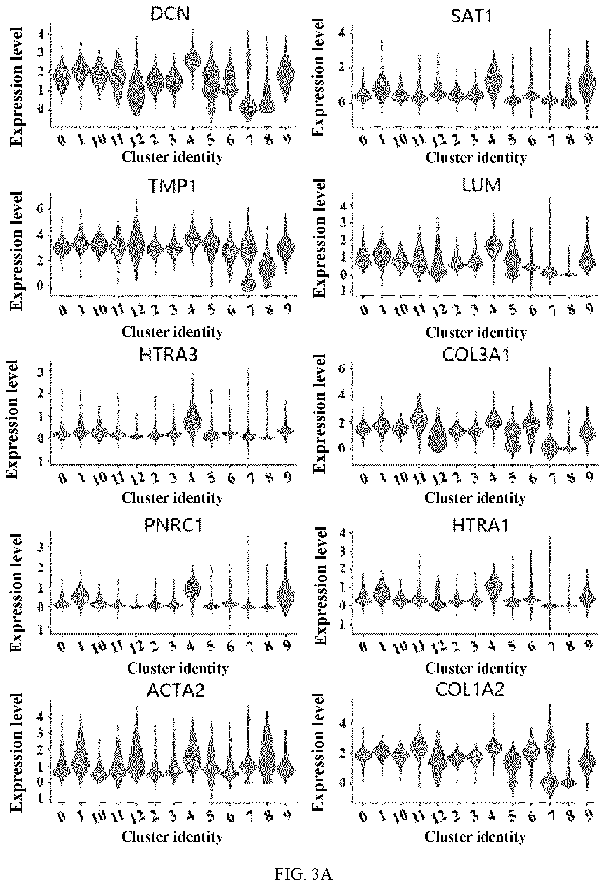

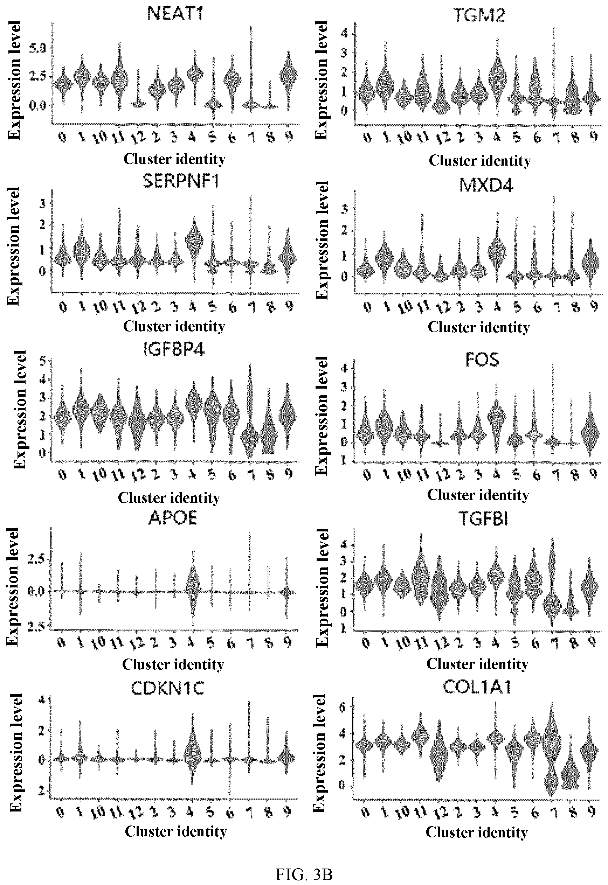

The expression levels of some of the marker genes of Hr-MSC listed in Example 2 were determined to obtain the expression of the marker genes in each cluster, as shown in FIGs. 3A and 3B.

Taking the marker gene DCN as an example, DCN was expressed in all clusters, but its expression was the highest in Cluster 4. The analysis of other figures and genes was the same as before. It can be known that the expression levels of the marker genes were different.

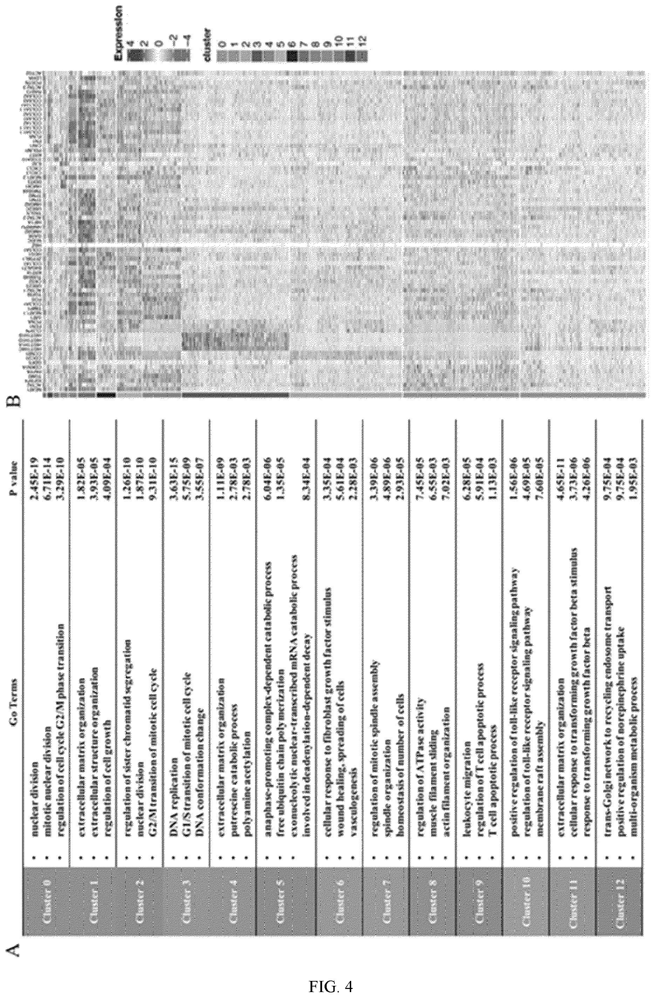

As shown in FIG. 4, after a series of standard quality controls and data preprocessing by Seurat, 13 cell clusters (Clusters 0-12) were obtained based on the characteristic genes of cells, and further analysis of the characteristic genes and functional enrichment of each cell cluster was performed to confirm cell identity. The results showed that Clusters 0, 2, 3, and 7, compared with other cell clusters, highly expressed known proliferation-related genes such as UBE2C, TOP2A, HMGB2, CDC20, PCNA, HIF1A, etc., suggesting that these clusters have strong proliferative capacity. The results of GO enrichment analysis indicated that these three clusters were also significantly enriched during cell nuclear division, DNA replication, and stem cell proliferation; Clusters 1, 4, and 11 had ACTA2, TGM2, FOS, TGFB1, FLNA, and COL3A1 as characteristic genes, which were mainly enriched in biological processes such as glucocorticoid response, pericyte differentiation, putrescine catabolism, extracellular matrix organization, and the like; Cluster 10 highly expressed S100A10, PDLIM1, and CAV1; Clusters 5, 8, and 12 were characterized by the high expression of UBE2S, TAGLN, TPM1, and TMSB4X, while the significant genes of Clusters 6 and 9 were mainly involved in immune regulation and wound repair-related pathways, and these genes include B4GALT1, HEG1, CXCL3, CXCL1, and HMOX 1.

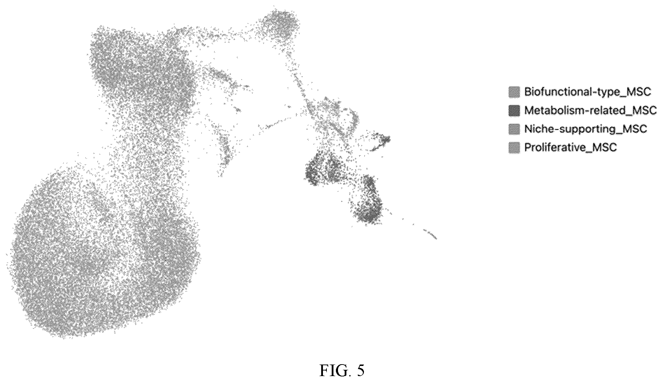

Ultimately, based on the gene expression profiles, functional similarity, and literature review, similar clusters were merged and named proliferative_MSC (composed of Clusters 0, 2, 3, and 7), niche-supporting _MSC (composed of Clusters 1, 4, and 11), metabolism-related_MSC (composed of Clusters 5, 8, and 12), and biofunctional type_MSC (functional stem cells; composed of Clusters 6 and 9, i.e., the CD29⁺/CD142⁺/S100A9⁺ Hr-MSC of the present application) (as shown in FIG. 5). Where Cluster 10 was not included in the subsequent analysis due to its small proportion and inability to be well classified into other clusters by GO enrichment.

According to the above results, combined with the single-cell data, CD29, CD142, and CYR61 of the biofunctional type MSC were selected as candidate markers for Hr-MSC; and CD29, CD142, and S100A9 of the biofunctional type MSC were selected as candidate markers for Hr-MSC, for subsequent experiments. The positive expression ratio of the above candidate markers in each cluster of Hr-MSC was at least 90%.

### Example 4: Sorting of CD29⁺/CD142⁺/CYR61⁺ Hr-MSC

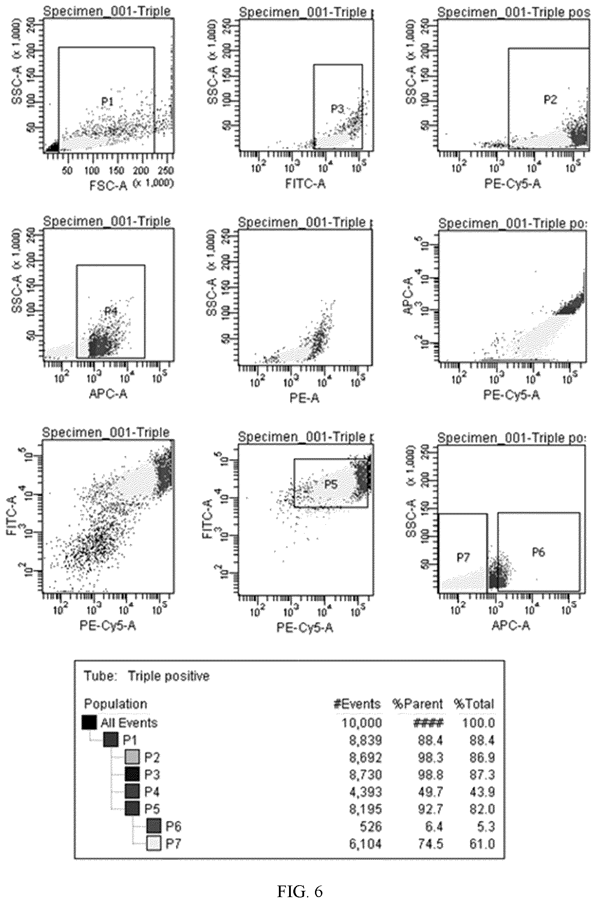

Referring to Example 1, in this example, single-cell transcriptomics sequencing analysis was utilized to obtain marker genes of the cluster with target therapeutic functions. Combined with the flow cytometry sorting technology, the target cluster was sorted from the primary mixed umbilical cord mesenchymal stem cells, as shown in FIG. 6. The cluster accounted for about 5.310% of the total cells. The cluster was identified by flow cytometry with positive markers: CD29⁺, CD142⁺, and CYR61⁺. The target cluster, which was positive for all three indexes, was designated as P6, accounting for 5.3% of the total stem cells, and was the target cluster used for subsequent amplification.

### Example 5: Sorting of CD29⁺/CD142⁺/S100A9⁺ Hr-MSC

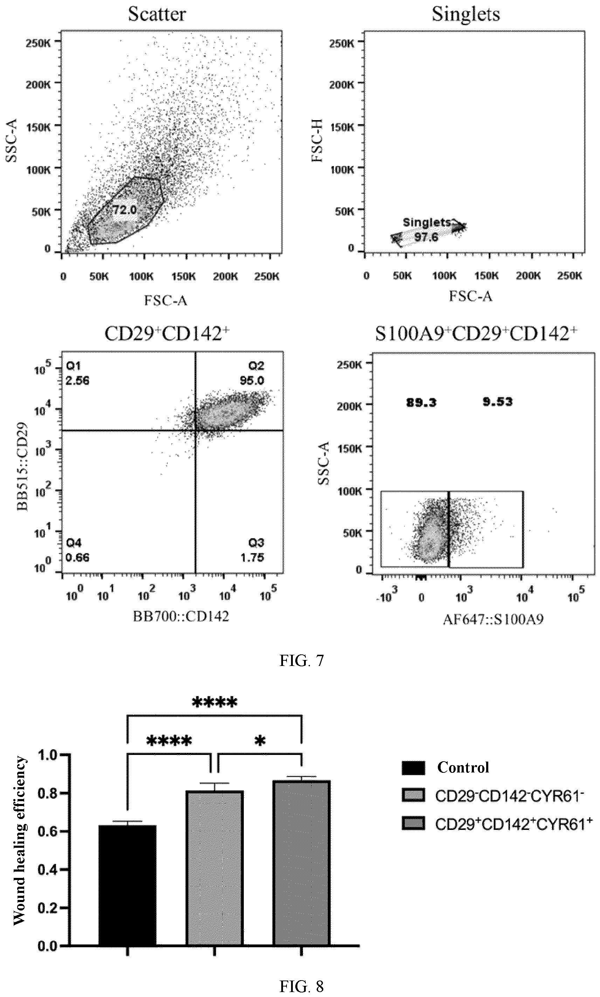

Referring to Example 1, in this example, single-cell transcriptomics sequencing analysis was utilized to obtain marker genes of the cluster with target therapeutic functions. Combined with the flow cytometry sorting technology, the target cluster was sorted from the primary mixed umbilical cord mesenchymal stem cells. The cluster was identified by flow cytometry with positive markers: CD29⁺, CD142⁺, and S100A9⁺. As shown in FIG. 7, the target cluster, which was positive for all three indexes, accounted for 9.53% of the total stem cells, and was the target cluster used for subsequent amplification.

### Example 6: Effect Example

In this example, full-thickness skin defects were made in adult zebrafish to assess the healing time (i.e., healing efficiency) with different administrations (i.e., the groups described below) on the defective wounds of adult zebrafish and validate the effectiveness (the model construction and effectiveness validation referenced Kennard Andrew S et al., Theriot Julie A.

(2021). Wounding Zebrafish Larval Epidermis by Laceration. Bio Protoc, 11(24), e4260; Mhlongo Fikile et al., Evaluation of the wound healing properties of South African medicinal plants using zebrafish and in vitro bioassays. [J]. J Ethnopharmacol, 2022, 286: 114867).

The adult zebrafish were kept in an automatic fish-keeping system at 28 °C with a cycle of 14 hours of light/10 hours of dark. They were fed once a day with newly hatched artemia. The zebrafish were divided into three groups: a control group (PBS, n = 6), a positive sorted MSC group (CD29⁺/CD142⁺/CYR61⁺ MSC, n = 6), and a negative sorted MSC group (CD29⁻/CD142⁻/CYR61⁻ MSC, n = 6).

The preliminary validation results, as shown in FIG. 8, indicated that both the positive sorted MSC group and the negative sorted MSC group exhibited significant differences in the wound healing efficiency compared to the control group (p < 0.001), and the wound healing efficiency of the positive sorted MSC group was significantly different from that of the negative sorted MSC group.

### Example 7: Effect Example

In this example, full-thickness skin defects were made in adult zebrafish to assess the healing time (i.e., healing efficiency) with different administrations (i.e., the groups described below) on the defective wounds of adult zebrafish and validate the effectiveness (the model construction and effectiveness validation referenced Kennard Andrew S et al., Theriot Julie A. (2021). Wounding Zebrafish Larval Epidermis by Laceration. Bio Protoc, 11(24), e4260; Mhlongo Fikile et al., Evaluation of the wound healing properties of South African medicinal plants using zebrafish and in vitro bioassays. [J]. J Ethnopharmacol, 2022, 286: 114867). The details are as follows:

The adult zebrafish were kept in an automatic fish-keeping system at 28 °C with a cycle of 14 hours of light/10 hours of dark. They were fed once a day with newly hatched artemia. The zebrafish were divided into four groups: a control group (PBS, n = 6), an unsorted MSC group (Wharton's jelly tissue MSC, n = 6), a positive sorted MSC group (CD29⁺/CD142⁺/S100A9⁺MSC, n = 6), and a negative sorted MSC group (CD29⁻/CD142⁻/S100A9⁻ MSC, n = 6). Then, the zebrafish were anesthetized using 0.03% triazine (Aladdin Biochemical Technology Co., Ltd., China). After anesthesia, full-thickness wounds (3 mm in diameter) were made on the left side of the back of the zebrafish using miniature ophthalmic scissors. Then, 10 µL of the prepared corresponding experimental solution (1 × 10⁴ cells) was subcutaneously injected into the area around the wound using a No. 33 syringe (Hamilton). After injection, the needle was carefully held in place for 5 min before the fish were placed back into the water in the circulatory system. If any leakage of the injected solution at the injection site was observed, euthanasia was performed on the fish, and they were excluded from the subsequent experiments. At 0, 5, 15, and 30 days post-wounding (dpw), the fish were anesthetized and then photographed. The wound area was measured using the ImageJ software.

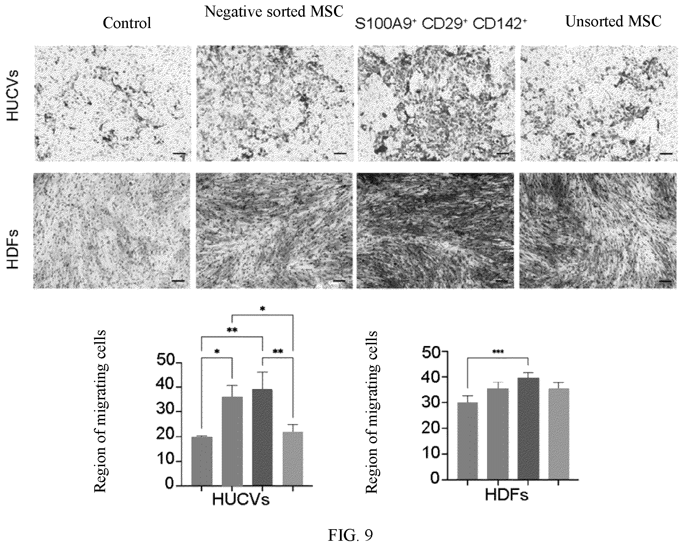

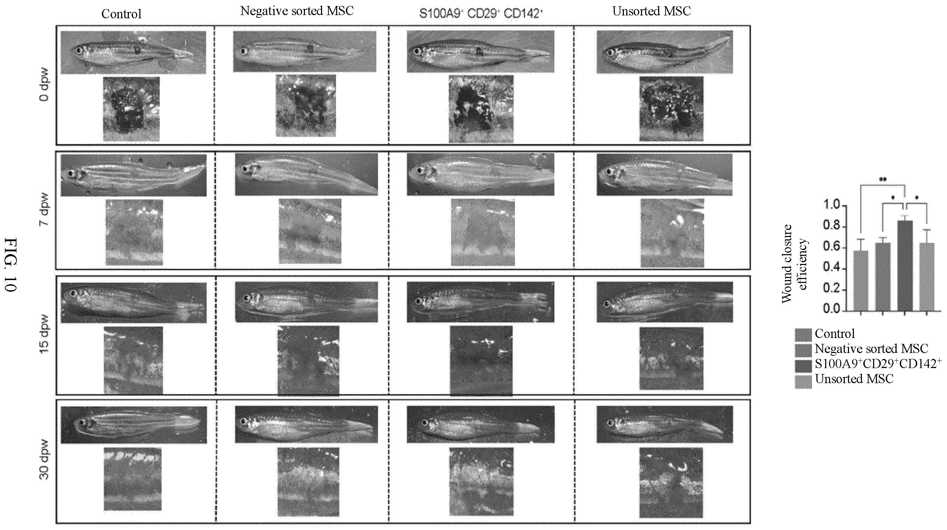

The results are shown in FIGs. 9 and 10, and indicated that a small amount of epithelial tissue was observed on the surface of the wounds at 5 days post-wounding, which gradually increased over time (FIG. 10). At 30 days post-wounding, the wounds in the control group were still relatively apparent with thin epithelial cell tissue; and different degrees of wound healing were observed in the unsorted group, positive sorted group, and negative sorted group. Notably, the positive sorted group showed more significant epithelialization, with good silver streak reconstruction, and melanin spots formed by melanocytes around the wound extending to the wound area. Specifically, the wound healing efficiency of the positive sorted group reached 0.857±0.050, while the wound healing efficiencies of the control group, negative sorted MSC group, and unsorted MSC group were 0.575±0.110, 0.648±0.129, and 0.646±0.129, respectively, and the positive sorted group showed a statistic difference in wound healing efficiency, indicating that CD29⁺/CD142⁺/S100A9⁺ MSC could better promote wound healing.

### Example 8: System for Identifying Wound Repair-Dominant Functional Cell Cluster

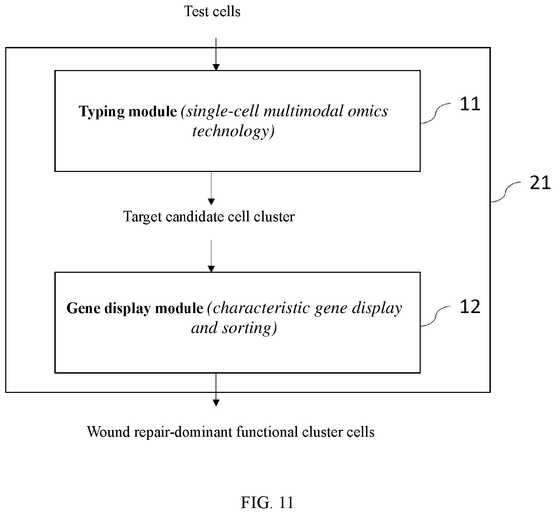

This example provides a system 21 for identifying a wound repair-dominant functional cell cluster, as shown in FIG. 11, which comprises: a typing module 11 and a gene display module 21.

Where the typing module 11 performed molecular typing on the test cells by the single-cell multimodal omics technology to obtain a target candidate cell cluster.

The gene display module 12 performed characteristic gene display and sorting on the target candidate cell cluster obtained by the typing module 11 to obtain a cell cluster comprising characteristic genes, and the obtained cell cluster was the wound repair-dominant functional cluster cell.

The characteristic genes expressed one or more of the following marker combinations:
(1) CD29, CD142, and CYR61;
(2) DCN, TGFB1, COL3A1, COLIA2, and COLIA1; and
(3) NEAT1, TMP1, GFBP4, SAT1, TGM2, LUM, SERPNF1, MXD4, FOS, ACTA2, HTRA3, HTRA1, PNRC1, APOE, and CDKN1C.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A biomarker combination for a wound repair-dominant functional cell, comprising CD29, CD142, and CYR61.

2. A biomarker combination for a wound repair-dominant functional cell, comprising CD29, CD142, and S100A9.

3. The biomarker combination according to claim 1 or 2, wherein the biomarker combination further comprises one or more selected from DCN, TGFB1, COL3A1, COLIA2, and COLIA1;
preferably, the combination further comprises one or more selected from NEAT1, TMP1, GFBP4, SAT1, TGM2, LUM, SERPNF1, MXD4, FOS, ACTA2, HTRA3, HTRA1, PNRC1, APOE, and CDKN1C.

4. A wound repair-dominant functional cell formulation, comprising a stem cell positive for CD29, CD142, and CYR61.

5. A wound repair-dominant functional cell formulation, comprising a stem cell positive for CD29, CD142, and S100A9.

6. The wound repair-dominant functional cell formulation according to claim 4 or 5, wherein the stem cell is a mesenchymal stem cell;
and/or, the stem cell expresses one or more biomarkers selected from DCN, TGFB1, COL3A1, COLIA2, and COLIA1.

7. The wound repair-dominant functional cell formulation according to claim 6, wherein the stem cell expresses one or more biomarkers selected from NEAT1, TMP1, GFBP4, SAT1, TGM2, LUM, SERPNF1, MXD4, FOS, ACTA2, HTRA3, HTRA1, PNRC1, APOE, and CDKN1C.

8. A cell cluster, comprising a stem cell positive for CD29, CD142, and CYR61, wherein
the proportion of the stem cell in the cell cluster is at least 90%.

9. A cell cluster, comprising a stem cell positive for CD29, CD142, and S100A9, wherein
the proportion of the stem cell in the cell cluster is at least 90%.

10. The cell cluster according to claim 8 or 9, wherein the stem cell is a mesenchymal stem cell;
and/or, the stem cell expresses one or more selected from DCN, TGFB1, COL3A1, COLIA2, and COLIA1.

11. The cell cluster according to claim 10, wherein the stem cell expresses one or more selected from NEAT1, TMP1, GFBP4, SAT1, TGM2, LUM, SERPNF1, MXD4, FOS, ACTA2, HTRA3, HTRA1, PNRC1, APOE, and CDKN1C.

12. A pharmaceutical composition, comprising a stem cell and a pharmaceutically acceptable carrier and/or excipient, wherein the stem cell is as described in any one of claims 4-7.

13. A pharmaceutical combination, comprising a first therapeutic agent and a second therapeutic agent, wherein the first therapeutic agent comprises the pharmaceutical composition according to claim 12;
preferably, the second therapeutic agent is selected from one or more of an immunosuppressant, an analgesic, and an anti-infective agent.

14. A method for promoting wound repair, comprising administering to a subject in need thereof the wound repair-dominant functional cell formulation according to any one of claims 4-7, the cell cluster according to any one of claims 8-11, the pharmaceutical composition according to claim 12, or the pharmaceutical combination according to claim 13.

15. A method for identifying a wound repair-dominant functional cell cluster, wherein the method comprises:
(1) performing molecular typing on cells in a test sample based on the single-cell multimodal omics technology to obtain a target candidate cell cluster; and
(2) performing characteristic gene display and sorting on the target candidate cell cluster obtained in (1) to obtain a cell cluster comprising characteristic genes, the cell cluster being a wound repair-dominant functional cell cluster;
the characteristic genes encode the biomarker combination according to any one of claims 1-3.

16. The method according to claim 15, wherein (1) is followed by (1-1): obtaining positioning information of the target candidate cell cluster through spatial transcriptomics, the positioning information comprising an enrichment condition and a positioning condition.

17. A system for identifying a wound repair-dominant functional cell cluster, comprising a typing module and a gene display module,
wherein the typing module performs molecular typing on test cells by the single-cell multimodal omics technology to obtain a target candidate cell cluster; the gene display module performs characteristic gene display and sorting on the target candidate cell cluster obtained by the typing module to obtain a cell cluster comprising characteristic genes, and the cell cluster is a wound repair-dominant functional cluster cell;
the characteristic genes encode the biomarker combination according to any one of claims 1-3.

18. A kit for identifying a wound repair-dominant functional cell cluster,
comprising a reagent for detecting the biomarker combination according to any one of claims 1-3.
